# EUROPEAN PATENT SPECIFICATION

(11) **EP 1 800 289 B1**
(45) Date of publication and mention of the grant of the patent: **23.05.2012**
(21) Application number: 04783582.2
(22) Date of filing: 09.09.2004
(51) Int. Cl.: G09G 5/00, G06T 3/00

(54) **SINGLE -PASS IMAGE WARPING SYSTEM AND METHOD WITH ANISOTROPIC FILTERING**
EINDURCHGANGS-BILDVERZERRUNGSSYSTEM UND -VERFAHREN MIT ANISOTROPER FILTERUNG
SYSTEME DE DEFORMATION D'IMAGE MONOPASSAGE ET METHODE FAISANT APPEL A UN FILTRAGE ANISOTROPIQUE

(43) Date of publication of application: 27.06.2007
(73) Proprietor: QUALCOMM Incorporated, San Diego, CA 92121 (US)
(72) Inventor: LACHINE, Vladimir, Toronto, Ontario M4Y 2P7 (CA); SMITH, Gregory L., Toronto, Ontario M6S 4K6 (CA); LEE, Louie, Richmond Hill, Ontario L4C 0C4 (CA)
(74) Representative: Hughes, Andrea Michelle
(86) International application number: PCT/US2004/029393
(87) International publication number: WO 2006/031213

(56) References cited:
- WO-A1-99/06952
- US-A1- 2004 012 603
- US-A1- 2004 113 921
- NED GREENE ET AL: "Creating Raster Omnimax Images from Multiple Perspective Views Using the Elliptical Weighted Average Filter" IEEE COMPUTER GRAPHICS AND APPLICATIONS, IEEE SERVICE CENTER, NEW YORK, NY, US, vol. 4, no. 6, 1 June 1986 (1986-06-01), pages 21-27, XP011155477 ISSN: 0272-1716
- MCCORMACK J; PERRY R; FARKAS K I; JOUPPI N P: "Feline: fast elliptical lines for anisotropic texture mapping" COMPUTER GRAPHICS PROCEEDINGS. SIGGRAPH 99, ACM, 2 PENN PLAZA, SUITE 701 - NEW YORK USA, 8 August 1999 (1999-08-08), pages 243-250, XP040102472
- SHIN H-C ET AL: "EFFICIENT EDGE FUNCTION BASED ANISOTROPIC TEXTURE FILTERING" IEICE TRANSACTIONS ON FUNDAMENTALS OF ELECTRONICS,COMMUNICATIONS AND COMPUTER SCIENCES, ENGINEERING SCIENCES SOCIETY, TOKYO, JP, vol. E87-A, no. 4, 1 April 2004 (2004-04-01), pages 964-970, XP001193284 ISSN: 0916-8508
- CHEN, B. ET AL.: 'Footprint Area Sampled Texturing' COMPUTER SCIENCE DEPARTEMENT TECHNICAL REPORT 001-2002, UNIVERSITY OF MINNESOTA 2004, pages 1 - 32, XP002984560
- ZWICKER, M. ET AL.: 'EWA Splatting' IEEE TRANS. ON VIS. AND COMP. GRAPHICS vol. 8, no. 3, July 2002 - September 2002, pages 223 - 239, XP002984561

## Description

### FIELD OF THE INVENTION

This invention relates to filtering methods in digital image transformation, and in particular, anisotropic filtering in signal resampling in single-pass digital image warping systems.

### BACKGROUND OF THE INVENTION

A digital image is basically a two-dimensional array of digital data with each entry representing a pixel of the digitized image. Pixel data contain intensity components like red, green, and blue (RGB). Display systems electronically assign intensity values to these different components based on input digital data. The displayed images, however, may have various optical and geometric distortions, such as lens distortions such as pincushion and barrel distortions, projection keystone distortions, and so on. One way to compensate for these distortions is digital image warping, or "non-uniform scaling". Image warping may be defined as a process of dynamically resampling a regularly spaced input image to produce an output image with different spacing than the input Image. Image warping is widely used in linear scaling operations like zoom and shrink, which are particular and simpler cases.

There are two basic stages in an image warping system: spatial transformation, and resampling.

A spatial transformation is defined as a mapping function between pixel coordinates in the input and output images. Inverse mapping, which specifies coordinates in the input image as a function of the coordinates in the output image, is usually used to cover the entire output image coordinate space and to avoid leaving "holes".

Resampling, on the other hand, is the process of using several input pixels surrounding an input pixel to interpolate the output image pixels. The neighborhood containing these input pixels, which participate in the interpolation, is called filter footprint. It is well known in the art that the larger the filter footprint, the better the output image quality. In addition, anisotropic footprints are known to yield better output image quality. There is a trade off between the size and anisotropy of the footprint and the efficiency of the hardware implementation. Resampling is accomplished by dynamically band limiting the spatial frequency of the warped image to the Nyquist limit in all directions around the processed output pixel.

In order to reduce hardware resources required for filtering, image warping can be conducted in two separate horizontal and vertical passes. In this case the transformation must be separable (i.e., capable of being decomposed), into a sequence of two orthogonal, one-dimensional transformations. Examples of two-pass warping are used in United States Patent Nos. 5,175,808, 5,594,676, and 6,061,477. The two-pass approach has many drawbacks however. Firstly, not all geometrical transformations are separable (bottleneck and fold over problems). Secondly, the separation itself may introduce geometrical distortions. Thirdly, two-pass filtering is prone to producing jagging artifacts, especially when the geometrical transformation has rotational component. Therefore, it is desirable to design a single-pass method for 2D image transformation (warping) that is free from disadvantages of the two-pass algorithm.

In multi-rate signal processing, in order to avoid unwanted artifacts, the resampling process of a digital image includes the following steps: reconstructing the continuous signal from its discrete samples, warping the continuous signal, low pass filtering the continuous signal to eliminate aliased frequencies above the normalized Nyquist frequency in the target image, and sampling the continuous warped signal at the output pixel grid sampling locations.

It is known in the art that these steps of the ideal resampling procedure may be concatenated into a single unified elliptical Gaussian filter called Elliptical Weighted Averaging [Paul S. Heckbert, "Fundamentals of Texture Mapping and Image Warping", master Thesis, UCB, 1989]. Since then many efforts have been made to reduce needed hardware in terms of memory and clocks by using approximations of the ellipse radii.

United States Patent No. 6,016,152, to Dickie, discloses apparatus and method for non-uniform image scaling approximated by corresponding affine transformation, which is used to define an ellipse in the source image. A linear transformation from a unit circle in the source image to the ellipse in the source image is calculated. A pixel value in the destination image is determined by identifying pixels included in the source image in order to reduce artifacts in non-uniform scaling applications.

United States Patent No. 6,005,582, to Gabriel et. al., discloses method and system for texture mapping images with anisotropic filtering that includes passing an interpolating filter along a line of anisotropy in a texture map and computing a weighted sum of the outputs of the interpolating filter to compute pixel intensity values. The weighting of these output values can be computed using a one-dimensional digital filter. The line of anisotropy is derived from the inverse transform, which describes the mapping of a point on the surface of a geometric primitive to a point in the texture map.

United States Patent No. 6,292,193, to Perry et. al., discloses techniques for anisotropic texture mapping using multiple space-invariant filtering operations per pixel. A circular pixel filter is projected onto a texture map to define an elliptical footprint in that texture map. Sample points are determined on a line in the footprint that closely approximates the major axis of the ellipse. These sample points are mapped to levels of detail and locations within mip-map. Using a space invariant filter, a texture value is computed for each sample point using data from one or more texture maps within mi-map. These texture values for the sample points are post-filtered using a Gaussian filter function and summed to produce a final texture value.

European Patent No. EP0920671B1, to Lohmeyer et. al., discloses method for computationally efficient single pass digital image warping that consists of the following stages: increasing the sampling rate of an input image above Nyquist rate, warping the upsampled Image using a low quality interpolator such as a bilinear interpolator, and downsampling the warped image to the same resolution as the input image. Downsampling and warping stages can be combined into one step by modifying the geometric transformation function when warping the upsampted image.

However, these and other prior art systems provide a modest trade-off between aliasing and sharpness because their Impulse responses produce pure frequency responses. Therefore, there is a need for an improved interpolation method and apparatus to support more flexible filtering in digital image warping System.

### SUMMARY OF THE INVENTION

A digital signal processing method for optimized hardware and software implementation of single-pass digital image resampling with an anisotropic filter to transform two-dimensional input image having an input pixel coordinate space and input digital pixel data, into an output image having an output pixel coordinate space and output digital pixel data, said method comprising: (a) obtaining two-dimensional input digital image data for an output pixel; (b) mapping the output pixel coordinates onto the input image coordinate space to determine a mapped coordinate position; (c)determining the Jacobian matrix of the output pixel coordinates with respect to the input coordinates at the mapped coordinate position determined in (b); (d) determining major and minor radii and the orientation of the major radius to uniquely determine an ellipse around the mapped coordinate position determined in (b), formed by two row vectors of the determined Jacobian matrix in (c), wherein the major radius of said ellipse is aligned alongside an edge orientation, and the lengths of the radii depend on the strength of the edge; (e) extending or reducing the radii of said ellipse to increase or reduce a filtering area; (f) determining a footprint based on the result of (e) in the input image coordinate space around said mapped coordinate position determined in (b), to include input pixels participating in filtering; (g) transforming the ellipse of (d) into a circle having a radius of one unit; (h) determining

Document "Creating Raster Omnimax Images from Multiple Perspective Views Using the Elliptical Weighted Average Filter", by Ned Greene and Paul S. Heckbert, IEEE Computer Graphics and Applications, June 1986, vol. 6, nr, 6, pages 21-27, discloses a mapping program that, to minimize aliasing during resampling, uses the Elliptical Weighted Average (EWA) filter, a space-variant filter developed by the authors that computes a weighted average over an arbitrarily oriented elliptical area. Document "Feline: fast elliptical lines for anisotropic texture mapping", by McCormack J et Al., Proceedings of SIGGRAPH 99, 26th International Conference on Computer Graphics and Interactive Techniques, 8-13 Aug. 1999, pg. 243 - 250, describes another texture filtering technique called Feline (for Fast Elliptical Lines), that, like other hardware anisotropic filtering algorithms, uses an underlying space-invariant (isotropic) filter with mip-mapped data. Document "Efficient edge function based anisotropic texture filtering", by Shin et Al., IEICE Transactions on Fundamentals of Electronics, Communications and Computer Sciences, April 2004, vol. E87-A, nr. 4, pg. 964 - 970, proposes anisotropic texture filtering based on edge function. coefficients of the anisotropic filter for all input pixels in the footprint from a circularly symmetric profile as a distance of each input pixel from a center of the circle having a radius of one unit; (i)determining the value of the output pixel by accumulating values of the input pixels inside the footprint with coefficients determined in (h); and (j)saving the value of the output pixel.

A digital signal processing system for optimized hardware and software implementation of real-time single-pass digital image resampling with an anisotropic filter to transform two-dimensional input image having an input pixel coordinate space and input digital pixel data, into an output image having an output pixel coordinate space and output digital pixel data, said system comprising the following stages: (a) an input interface for obtaining two-dimensional input digital image data for an output pixel; (b) a coordinates generator (42) for mapping the output pixel coordinates onto the input image coordinate space to determine a mapped coordinate position; (c) a local scale estimator (114), coupled to said input interface and said coordinate generator, for determining the Jacobian matrix of the output pixel coordinates with respect to the input coordinates at the mapped coordinate position; (d)said local estimator, being further adapted for determining the major and minor radii and the orientation of the major radius to uniquely determine an ellipse around the mapped coordinate position, formed by two row vectors of the determined Jacobian matrix in (c), and align the major radius of said ellipse alongside an edge orientation and set the lengths of the radii based on a strength of the edge; (e) said local scale estimator, being further adapted to extend or reduce the radii of said ellipse to increase or reduce a filtering area; (f)a footprint generator (122), coupled to said local scale estimator and said coordinates generator, for determining a footprint in the input image coordinate space around the mapped coordinate position, to include input pixels participating in filtering; (g) a filter coefficients generator (124), coupled to said footprint generator and said local scale estimator, to transform the ellipse of (d) into a circle having a radius of one unit; (h) said filter coefficient generator, being further adapted to determine coefficients of the anisotropic filter for input pixels in the footprint from a circularly symmetric profile as a function of a distance of each input pixel from a center of the circle having a radius of one unit; (i) a filter (126), coupled to said filter coefficient generator and said footprint generator, to determine the value of the output pixel by accumulating values of the input pixels inside the footprint with coefficients determined in (h); and (j)an output interface, coupled to said filter, to save the value of the output pixel.

One example implementation of the present invention provides a method for generating a circularly symmetric profile on an anisotropic filter to produce sharpness and detail enhancement in an image.

In one example of the present invention, the filter's footprint has an elliptical shape extended by some factor, which establishes trade-off between image quality and processing speed.

In another example of the present invention, the filter's footprint has a fixed rectangular shape in order to minimize the complexity and bandwidth of a caching subsystem in the image warping system.

In yet another example of the present invention, the shape of the obtained ellipse is adjusted in accordance with local edge orientation.

Further details of different aspects and advantages of the embodiments of the invention will be revealed in the following description along with the accompanying drawings.

### BRIEF DESCRIPTION OF DRAWINGS

The accompanying drawings:
FIG. 1 is a block diagram illustrating a preferred embodiment of the image warping system built in accordance with the present invention;
FIG. 2 is a graphical representation of an example of mapping an output pixel from output image space into an ellipse in the input image space;
FIG. 3 is a graphical representation of anelliptical filter with deployed circularly symmetric profile over elliptical or rectangular footprint;
FIG. 4A is a graphical representation of the two-dimensional impulse response of the anisotropic filter with circularly symmetric profile;
FIG. 4B is a graphical representation of the two-dimensional frequency response of the anisotropic filter with circularly symmetric profile;
FIG. 5A is a graphical representation of the flow logic executed by the image warping system of FIG. 1; and
FIG. 5B is a graphical representation of the flow logic of an example of a system build in accordance with the present invention and using surface fitting to generate three invented parameters of a linear transformation from an ellipse to unit circle.

### DETAILED DESCRIPTION OF THE INVENTION

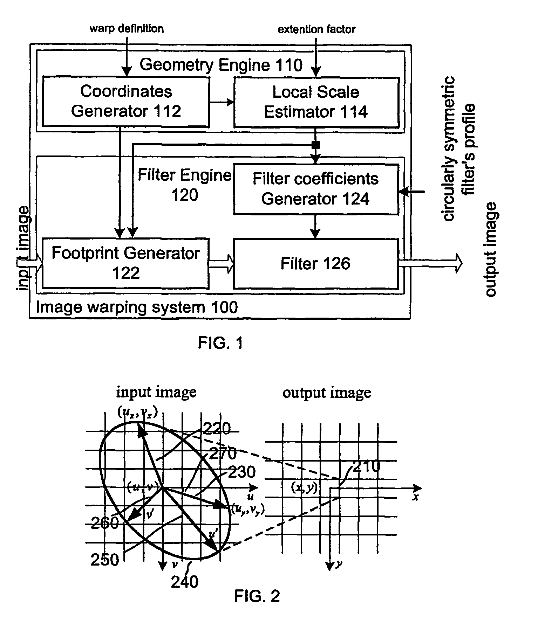

Reference is first made to FIG. 1 which shows an image warping system **100** made in accordance with a preferred embodiment of the invention. Image warping system **100** is a single-pass digital image warping system with circularly symmetric anisotropic filtering. Image warping system **100** includes a coordinates generator **112**, a local scale estimator **114**, a footprint generator **122**, a filter coefficients generator **124**, and a filter **126**. The coordinates generator **112** transforms pixel coordinates from an output image space onto an input image space. Local scale estimator **114**, calculates parameters of a linear transformation to a unit circle from an ellipse that approximates non-uniform image scaling function in the mapped output pixel. Footprint generator **122** determines input pixels to be participated in the filtering. Filter coefficients generator **124** calculates weights of the input pixels in the footprint using circularly symmetric profile. Finally, filter **126** calculates the output pixel.

Image warping system **100** includes two major units: a geometry engine **110** and a filter engine **120**. The function of geometry engine **110** relates to geometric transformations.

Geometry engine **110** includes coordinates generator **112** and local scale estimator **114**. Coordinates generator **112** maps the coordinates of an output pixel onto the input image coordinate space. This transformation could range from a straightforward scaling to a complicated warping. Mapping output coordinates onto input coordinates, or the so-called inverse transformation, has many advantages known to people skilled in the art, including covering the entire output pixel space and not leaving "holes" in the output image.

Another element of geometry engine **110** is local scale estimator **114**, which is crucial to the operation of filter engine **120** in calculating filter coefficients, as will be explained in more detail.

Local scale estimator **114** calculates elements of a 2x2 matrix that determines a linear transformation from an ellipse to a circle of radius one. This transformation approximates a non-uniform image scaling function in the position of the mapped output pixel and it is calculated based on the Jacobian matrix, which is a matrix containing first-order partial derivatives of a vector function in its rows. As illustrated in FIG. 2, the Jacobian matrix is a local affine approximation of the local mapping function at the position of the mapped output pixel **210:** $J \left(x, y\right) = \left[\begin{matrix}{u}_{x} & {v}_{x} \\ {u}_{y} & {v}_{y}\end{matrix}\right] = \left[\begin{matrix}\frac{\partial u}{\partial x} \left(x, y\right) & \frac{\partial v}{\partial x} \left(x, y\right) \\ \frac{\partial u}{\partial y} \left(x, y\right) & \frac{\partial v}{\partial y} \left(x, y\right)\end{matrix}\right] .$

The rows of the Jacobian matrix form two vectors **220** and **230** that determine the ellipse **240** with major radius **250,** minor radius **260,** and angle **270** between major radius and horizontal direction.

The ellipse **240** is characterized with a *2x2* transformation matrix based on the Jacobian matrix. Since three parameters are enough to describe an arbitrarily oriented ellipse (for example, two orthogonal radii and the orientation angle of the major radius define the ellipse), this matrix may be calculated in such a way that only three of its elements are non-zero and the fourth element is always zero.

In one example of the invention, local scale estimator **114** computes elements of the matrix *M* to implement the following linear transformation: $\left[\begin{matrix}uʹ \\ vʹ\end{matrix}\right] = \left[\begin{matrix}{M}_{00} & {M}_{01} \\ {M}_{10} & {M}_{11}\end{matrix}\right] ⁢ \left[\begin{matrix}u \\ v\end{matrix}\right] ,$
where (*u*,*v*) are source pixel coordinates relative to the center of the ellipse, and (*u*',*v*') are "ellipse space" coordinates. In the ellipse space, coordinates of the pixels inside the ellipse satisfy the equation: ${r}^{2} \left(uʹ, vʹ\right) = {\left(uʹ\right)}^{2} + {\left(vʹ\right)}^{2} \leq 1.$

Accordingly, matrix *M* maps the ellipse onto a circle of radius one. To generate this matrix, first the ellipse's major radius λ₁, minor radius λ₂ and angle α₁ of the major radius with the horizontal direction are found from the Jacobian matrix. Then, any required radii limitations are applied. Finally, three non-zero elements of *M* are calculated from these three parameters.

Local scale estimator **114** uses the rows of the Jacobian matrix *J* to solve the following equation: $Q = {J}^{- 1} ⁢ {\left({J}^{- 1}\right)}^{T} .$

Solving the above equation for *J*, under the constraint that the basis is orthogonal, i.e.: $J = \left[\begin{matrix}{λ}_{1} & 0 \\ 0 & {λ}_{2}\end{matrix}\right] ⁢ \left[\begin{matrix}cos {α}_{1} & sin {α}_{1} \\ - sin {α}_{1} & cos {α}_{1}\end{matrix}\right] ,$
eigenvectors of the Jacobian can be computed. In one example of the invention, this is done by first calculating the following intermediate variables from the elements of the Jacobian matrix: $A = {v}_{x}^{2} + {v}_{y}^{2} , B = - 2 ⁢ \left({u}_{x}⁢{v}_{x}+{u}_{y}⁢{v}_{y}\right) , C = {u}_{x}^{2} + {u}_{y}^{2} , F = {\left({u}_{x}⁢{v}_{y}-{u}_{y}⁢{v}_{x}\right)}^{2} ,$ $P = A - C , Q = A + C , T = sign \left(P\right) ⁢ \sqrt{{P}^{2} + {B}^{2}} .$

Then the lengths of the orthogonal radii of the ellipse, which are the required eigenvalues, are represented by the values: ${λ}_{1} = max ⁢ \left(\sqrt{\frac{2 ⁢ F}{Q + T}}, \sqrt{\frac{2 ⁢ F}{Q - T}}\right) , {λ}_{2} = min ⁢ \left(\sqrt{\frac{2 ⁢ F}{Q + T}}, \sqrt{\frac{2 ⁢ F}{Q - T}}\right) .$

Finally, the angle of the major radius with respect to the horizontal direction is calculated as follows: $tan {α}_{1} = sign \left(B\right) ⁢ sign \left(P\right) { \begin{matrix}\sqrt{\frac{T - P}{T + P}} , & {λ}_{1} = \sqrt{\frac{2 ⁢ F}{Q + T}} \\ - \sqrt{\frac{T + P}{T - P}} , & otherwise\end{matrix} ,$
or: $sin 2 ⁢ {α}_{1} = - \frac{P}{T} and cos ⁢ 2 ⁢ {α}_{1} = - \frac{B}{T} .$

In a particular example of the invention, the obtained ellipse may be extended by some factor to provide better flexibility in terms of image quality and processing speed. For example, if the input and output images are small enough, one can increase the footprint by some factor (i.e. perform filtering over a greater area). This will improve image quality because of better frequency response of the two-dimensional filter. On the other hand, the footprint may be reduced down to a minimal size in the case of large input and output images or big local shrink scales.

In addition, in order to restrict the number of processed pixels in a case of local shrink as a result of the warp map, two limits are imposed on λ₁ and λ₂. The first limit λₘₐₓ bounds maximum length of the ellipse radii and the second βₘₐₓ limits the anisotropy ratio, i.e. a ratio of the major to minor radii. Moreover, if either of λ₁,λ₂ is less than a predetermined minimum, it is replaced by the third limit λₘᵢₙ, which obviously should not be smaller than one. This last limit ensures that the filter footprint is never too small to perform interpolation. This limiting process may be expressed by the following equations: ${λ}_{1} ← cut \left({λ}_{min}, {λ}_{1}, {λ}_{max}\right) , {λ}_{2} ← cut \left({λ}_{min}, {λ}_{2}, {λ}_{max}\right) , {λ}_{1} ← min \left({λ}_{1},{λ}_{2}⁢{β}_{max}\right) .$

In one example of the invention, if, after imposing the limits, both radii are equal to one, which means that there is a local expansion in all directions, the ellipse's radii are adjusted according to the orientation of the local edge. The minor radius is kept as one and the major radius is extended proportionally to the strength of the edge.

The calculation of the matrix *M* is contrived so that *M*₁₀ is zero. This is done by concatenating onto the matrix *M* a suitable rotation in (*u*',*v*') space, which does not affect the radius. The mapping to the "ellipse space" (*u*',*v*') by means of *M* can be expressed as: $M = \left[\begin{matrix}cos θ & sin θ \\ - sin θ & cos θ\end{matrix}\right] ⁢ \left[\begin{matrix}\frac{cos {α}_{1}}{{λ}_{1} ⁢ {R}_{u}} & \frac{sin {α}_{1}}{{λ}_{1} ⁢ {R}_{u}} \\ - \frac{sin {α}_{1}}{{λ}_{2} ⁢ {R}_{u}} & \frac{cos {α}_{1}}{{λ}_{2} ⁢ {R}_{u}}\end{matrix}\right] ,$
where *Rᵤ* is the extension factor. The right-hand matrix rotates the coordinate space by the angle of the ellipse's major axis, then scales it non-uniformly in the axes directions so that the ellipse itself maps onto the unit circle. The left-hand matrix provides an additional rotation by θ. Since the left-hand rotation has no effect on filter's coefficient calculations, we can choose it arbitrarily to force *M*₁₀ to zero, i.e.: $tan θ = \frac{{λ}_{1} ⁢ sin α}{{λ}_{2} ⁢ cos α} or sin ⁢ θ = \frac{{λ}_{1} ⁢ sin {α}_{1}}{R} , cos θ = \frac{{λ}_{2} ⁢ cos {α}_{1}}{R} , where R = \sqrt{{{λ}_{2}}^{2} ⁢ {cos}^{2} ⁢ {α}_{1} + {{λ}_{1}}^{2} ⁢ {sin}^{2} ⁢ {α}_{1}} ⋅$
Substituting these values and replacing β =λ₁/λ₂ we finally obtain $M = \frac{1}{{R}_{u} ⁢ \sqrt{{λ}_{1} ⁢ {λ}_{2}}} ⁢ \frac{1}{\sqrt{\frac{{β}^{2} + 1}{2 ⁢ β} - \frac{{β}^{2} - 1}{2 ⁢ β} ⁢ cos 2 ⁢ {α}_{1}}} ⁢ \left[\begin{matrix}\frac{{β}^{2} + 1}{2 ⁢ β} - \frac{{β}^{2} - 1}{2 ⁢ β} ⁢ cos 2 ⁢ {α}_{1} & - \frac{{β}^{2} - 1}{2 ⁢ β} ⁢ sin 2 ⁢ {α}_{1} \\ 0 & 1\end{matrix}\right] .$

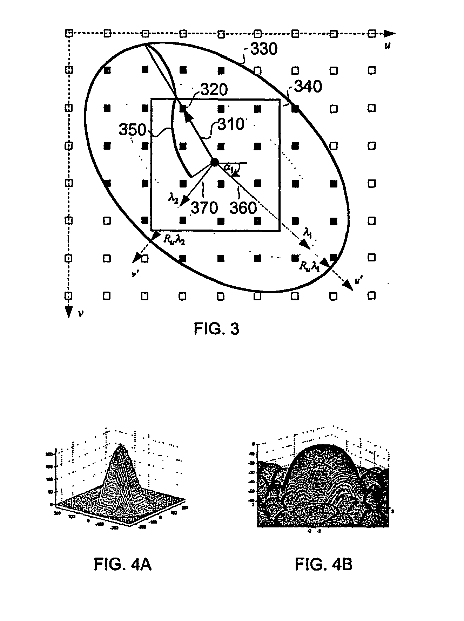

As shown in FIG. 3, a radius **310** of the input pixel **320**, inside the elliptical footprint **330** or rectangular footprint **340**, is considered as an index into a look-up table with profile (impulse response) **350** of a circularly symmetric filter. The profile of the circularly symmetric filter is stretched or shrunken in accordance with a distance from a center of the ellipse to its edge in a direction of input processed pixel. In this figure, λ₁ is a major radius **360**, λ₂ is a minor radius **370** and *Rᵤ* is the ellipse extension factor. If λ₁ = λ₂ = 1, then the filter performs upsampling in all directions. If λ₁ > 1,λ₂>1, then the filter performs downsampling in all directions. But, if λ₁>1,λ₂=1, then there is a mixture of upsampling and downsampling with a smooth transition between expansion and shrinking.

In a case of fixed extension factor *Rᵤ* and fixed number of coefficients *N* in the impulse response, the circularly symmetric profile has variable length and variable fractional bandwidth that depend on the ellipse orientation and shape. Two-dimensional impulse and frequency responses of the filter for the particular case of *R*ᵤ = 2 and *N* = 256 are shown in FIG. 4A and FIG. 4B respectively.

In case of anisotropic elliptical filtering, filter engine **120** needs at least five parameters to generate warped image: two coordinates *u*,*v* to map output pixel coordinates to the input image coordinate space and three parameters *M*₀₀,*M*₀₁,*M*₁₁ to represent a linear transformation from an ellipse to a unit circle.

Since for some spatial transformations the first two maps may be very complicated or even may not have analytical form, in one particular example of the invention, these maps are approximated by surfaces fitted with polynomials: $u \left(x, y\right) = {\sum }_{i = 0}^{I} {\sum }_{j = 0}^{J} {a}_{ij} ⁢ {x}^{i} ⁢ {y}^{j} , v \left(x, y\right) = {\sum }_{i = 0}^{I} {\sum }_{j = 0}^{J} {b}_{ij} ⁢ {x}^{i} ⁢ {y}^{j} ,$
where *aᵢⱼ,bᵢⱼ* are polynomial coefficients, and *I*,*J* are degrees of the polynomials. This representation is abstracted from a physical model of the spatial transformation and, as a benefit, has a compact representation of the mapping functions.

In one example of the invention, the elements of the matrix *M*, which represent a linear transformation from an ellipse to a unit circle, are calculated directly from the coordinate polynomials in real time. They are calculated from the Jacobian matrix, the four elements of which are partial derivatives of the two coordinate polynomials.

In another example of the invention, the elements of the matrix *M* are represented by polynomial fitted surfaces, in order to save in computation time, as follows: ${M}_{00} \left(x, y\right) = {\sum }_{i = 0}^{I} {\sum }_{j = 0}^{J} {c}_{ij} ⁢ {x}^{i} ⁢ {y}^{j} , {M}_{01} \left(x, y\right) = {\sum }_{i = 0}^{I} {\sum }_{j = 0}^{J} {d}_{ij} ⁢ {x}^{i} ⁢ {y}^{j} , {M}_{11} \left(x, y\right) = {\sum }_{i = 0}^{I} {\sum }_{j = 0}^{J} {e}_{ij} ⁢ {x}^{i} ⁢ {y}^{j} ,$
where *cᵢⱼ*,*dᵢⱼ*,*eᵢⱼ* are polynomial coefficients, and *I*,*J* are orders of the polynomials.

Footprint generator **122** defines an area around the mapped position of the output pixel in the input image coordinate space to include input pixels for generating the output pixel data. In one example of the invention, the footprint has an elliptical shape based on computed elements of the matrix M. This example provides better image quality but demands more hardware resources. In another example of the invention, the footprint has a rectangular shape in order to minimize the complexity of a pixel inclusion while compromising on output image quality.

Filter coefficients generator **124** evaluates weights of the input pixels included in the footprint. In one example of the invention, the weight of an input pixel is a function of its distance from the center of the ellipse mapped onto a circle of radius one. In one particular implementation of this example, these weights are calculated dynamically for higher flexibility at the expense of hardware resources. In another implementation, in order to save on hardware resources, the weights are taken from a look-up table with pre-calculated circularly symmetric profile.

To understand the method of generating the profiles, it is necessary to mention that there are four standard approaches to design a two-dimensional finite impulse response (FIR) filter: the window method, the frequency sampling method, the frequency transformation method, and the optimal filter design. The window method, which is a straightforward extension of a one-dimensional filter design, has been chosen, in one example of the invention, for the design of circularly symmetric filter profile.

If the frequency response *H*(ω*ᵤ*,ω*ᵥ*) is known, one can determine the impulse response of the filter *h*(*u*',*v*') by inverse Fourier transformation. The required finite impulse response (FIR) filter is obtained by truncating the impulse response *h*(*u*',*v*') and multiplying it by a window function *w*(*u*',*v*'), so that *h_{d}* (*u'*,*v'*) = *h*(*u*',*v*')*w*(*u*',*v*')

A desirable frequency response for image warping is a circularly symmetrical ideal lowpass filter $H \left({ω}_{u}, {ω}_{v}\right) = { \begin{matrix}1 , & {ω}_{u}^{2} + {ω}_{v}^{2} \leq {ω}_{c}^{2} \\ 0 , & otherwise\end{matrix} , where - π \leq {ω}_{u} , {ω}_{v} \leq π .$

An impulse response of such a filter with cutoff frequency of ω*_{c}* is given by $h \left(uʹ, vʹ\right) = \frac{{ω}_{c}}{2 ⁢ πr \left(uʹ, vʹ\right)} ⁢ {J}_{1} \left({ω}_{c}⁢r\left(uʹ, vʹ\right)\right) ,$
where $r \left(uʹ, vʹ\right) = \frac{{R}_{u} ⁢ \sqrt{{\left(uʹ\right)}^{2} + {\left(vʹ\right)}^{2}}}{N} = \frac{{R}_{u} ⁢ n}{N}$ is the normalized radius, *n* satisfies 0≤*n*<*N* , *J*, is the Bessel function of the first kind and first order. The two-dimensional window *w*(*u*',*v*') is based upon a one-dimensional Kaiser window prototype. With the rotated formulation the actual region of support for the design will be a circular subregion of *Rᵤ*. $w \left(uʹ, vʹ\right) = { \begin{matrix}\frac{{I}_{0} ⁢ \left(β⁢\sqrt{1 - {r}^{2} \left(uʹ, vʹ\right) / {R}_{u}^{2}}\right)}{{I}_{0} \left(β\right)} , & r \left(uʹ, vʹ\right) \leq {R}_{u} \\ 0 , & otherwise\end{matrix} ,$
where *I*₀() is the modified Bessel function of the first kind, order zero, β is a parameter.

Visual appearance of the output warped image may be significantly improved by emphasizing its high frequency contents to enhance the edges and details therein. Filter coefficients generator **124**, in one example of the invention, uses the classic linear unsharp masking algorithm for this purpose. In this technique a fraction of the high-pass filtered image is added to the image itself. In one modification of the linear unsharp masking algorithm, when low-passed filtered (smoothed) signal over pixel's neighborhood is subtracted from the pixel's value, the enhanced image *O*(*x*, *y*) is obtained from the input image *I*(*x*, *y*) as follows: $O \left(x, y\right) = I \left(x, y\right) + α ⁢ \left(I\left(x, y\right)-{I}_{s}\left(x, y\right)\right) = \left(1+α\right) ⁢ I \left(x, y\right) - α ⁢ {I}_{s} \left(x, y\right) ,$
where *I*(*x*, *y*) and *O*(*x*, *y*) are input and output pixel values, *Iₛ(x, y)* is a smoothed pixel value over some neighborhood, and α is a factor of enhancement. Here, α < 0 implies smoothing, α = 0 implies bypass, and α > 0 implies sharpening.

In a case of digital image warping, this formula is transforming to the following $O \left(x, y\right) = \left(1+α\right) ⁢ {I}_{w} \left(x, y\right) - α ⁢ {I}_{s} \left(x, y\right) ,$
where *I_{w}*(*x*,*y*) is a warped pixel value and *Iₛ*(*x*,*y*) is a smoothed pixel value in the output space. The footprint for calculating the warped and smoothed pixel values is the same. In order to save on hardware resources the two operations are combined in one circularly symmetric profile, which assumes both filtering terms. These terms take the form: $O \left(x, y\right) = \left(1+α\right) ⁢ \frac{{\sum }_{n = 0}^{N \left(x, y\right)} {k}_{n}^{w} ⁢ I \left({u}_{n}, {v}_{n}\right)}{{\sum }_{n = 0}^{N \left(x, y\right)} {k}_{n}^{w}} - α ⁢ \frac{{\sum }_{n = 0}^{N \left(x, y\right)} {k}_{n}^{s} ⁢ I \left({u}_{n}, {v}_{n}\right)}{{\sum }_{n = 0}^{N \left(x, y\right)} {k}_{n}^{s}} = \frac{{\sum }_{n = 0}^{N \left(x, y\right)} \left(\left(1+α\right)⁢{k}_{n}^{w}-α⁢\frac{{\sum }_{n = 0}^{N \left(x, y\right)} {k}_{n}^{w}}{{\sum }_{n = 0}^{N \left(x, y\right)} {k}_{n}^{s}}⁢{k}_{n}^{s}\right) ⁢ I \left({u}_{n}, {v}_{n}\right)}{{\sum }_{n = 0}^{N \left(x, y\right)} {k}_{n}^{w}} .$
where ${k}_{n}^{w}$ are warping filter's coefficients, ${k}_{n}^{s}$ are smoothing filter's coefficients, and index *n* counts input pixels (*uₙ*, *vₙ*) belonging to the footprint. The number of input pixels *N*(*x*,*y*) in the footprint is a function of output pixel coordinates. So, we have a new set of coefficients ${k}_{n} = \left(1+α\right) ⁢ {k}_{n}^{w} - α ⁢ \frac{{\sum }_{n = 0}^{N \left(x, y\right)} {k}_{n}^{w}}{{\sum }_{n = 0}^{N \left(x, y\right)} {k}_{n}^{s}} ⁢ {k}_{n}^{s}$
that assume both smoothing and warping components in the filter for detail enhancement of the output image, where ${\sum }_{n = 0}^{N \left(x, y\right)} {k}_{n} = {\sum }_{n = 0}^{N \left(x, y\right)} {k}_{n}^{w} .$

As seen from the last equation, this approach for detail enhancement can be applied if a ratio of coefficient sums over the same footprint is a constant for all output pixels of the warp, i.e. $T \left(x, y\right) = \frac{{T}^{w} \left(x, y\right)}{{T}^{s} \left(x, y\right)} = \frac{{\sum }_{n = 0}^{N \left(x, y\right)} {k}_{n}^{w}}{{\sum }_{n = 0}^{N \left(x, y\right)} {k}_{n}^{s}} = T .$
For an elliptical footprint, the constant *T* may be estimated by the following equation: $T \approx \frac{{\sum }_{i = 0}^{{N}^{w} - 1} {s}_{i} ⁢ {k}_{i}^{w}}{{\sum }_{i = 0}^{{N}^{s} - 1} {s}_{i} ⁢ {k}_{i}^{s}} ,$
where *i* counts filter's taps, *s*ᵢ is an area of an elliptical ring occupied by *i* - *th* coefficient, *N^{w}* is the number of taps in warping part of the filter and *N^{z}* is the number of taps in smoothing part of the filter.

If λ₁,λ₂ are major and minor radii of the elliptical footprint, then ${s}_{i} = π ⁢ \frac{{λ}_{1} ⁢ \left(i+1\right) ⁢ {R}_{u}}{N} ⁢ \frac{{λ}_{2} ⁢ \left(i+1\right) ⁢ {R}_{u}}{N} - π ⁢ \frac{{λ}_{1} ⁢ i ⁢ {R}_{u}}{N} ⁢ \frac{{λ}_{2} ⁢ i ⁢ {R}_{u}}{N} = \frac{π ⁢ {λ}_{1} ⁢ {λ}_{2} ⁢ {R}_{u}^{2}}{{N}^{2}} ⁢ \left({\left(i+1\right)}^{2}-{i}^{2}\right) = \frac{π ⁢ {λ}_{1} ⁢ {λ}_{2} ⁢ {R}_{u}^{2}}{{N}^{2}} ⁢ \left(2⁢i+1\right)$
and ${R}_{u} = max \left({R}_{u}^{w}, {R}_{u}^{s}\right) , {N}^{w} = \frac{N ⁢ {R}_{u}^{w}}{{R}_{u}} , {N}^{s} = \frac{N ⁢ {R}_{u}^{s}}{{R}_{u}} ,$ where *N* is the maximum number of the filter taps.

After substituting we finally get: $T = \frac{{T}^{w}}{{T}^{s}} = \frac{{\sum }_{i = 0}^{{N}^{w} - 1} π ⁢ \frac{{λ}_{1} ⁢ {λ}_{2} ⁢ {R}_{u}^{2}}{{N}^{2}} ⁢ \left(2⁢i+1\right) ⁢ {k}_{i}^{w}}{{\sum }_{i = 0}^{{N}^{s} - 1} π ⁢ \frac{{λ}_{1} ⁢ {λ}_{2} ⁢ {R}_{u}^{2}}{{N}^{2}} ⁢ \left(2⁢i+1\right) ⁢ {k}_{i}^{s}} = \frac{{\sum }_{i = 0}^{N - 1} \left(2⁢i+1\right) ⁢ {k}_{i}^{w}}{{\sum }_{i = 0}^{N - 1} \left(2⁢i+1\right) ⁢ {k}_{i}^{s}} .$
If *N^{w}* > *N^{s}*, then ${k}_{i}^{s} = 0$ for *N^{s}*≤*i*<*N^{w}*, and if *N^{s}*>*N^{w}* then ${k}_{i}^{w} = 0$ for *N^{w}*≤*i*<*N^{s}*.

As we can see from the last equation, for an elliptical footprint the ratio *T* depends only on corresponding coefficients of warping and smoothing filters. This means that filter design of the present invention, in this particular implementation, is appropriate for image detail enhancement.

If the circularly symmetric profile is a function of the squared radius instead of the radius itself, then the formula for the calculation of the constant *T* is reduced to the following: $T = \frac{{\sum }_{i = 0}^{N - 1} {k}_{i}^{w}}{{\sum }_{i = 0}^{N - 1} {k}_{i}^{s}} .$

Once the weights of the input pixels are calculated, filter **126** generates the output pixel by accumulating the input pixel intensities in the footprint. Image warping system **100** then moves on to process the next output pixel until the whole output image is generated.

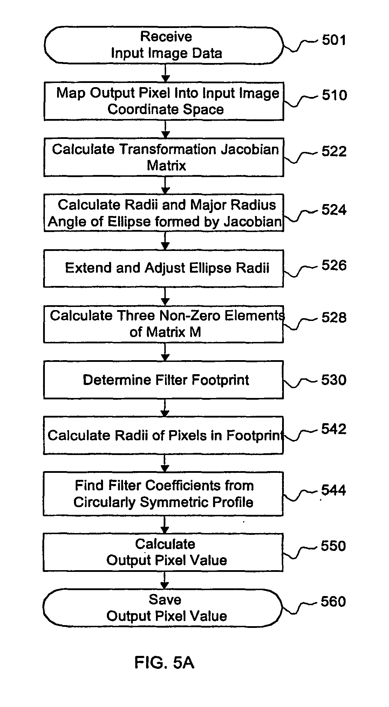

FIG. 5A represents the logic flow of an example of image warping system **100** built according to the present invention. At step **501** the system receives digital data corresponding to an input image. At step **510**, the coordinates of an output pixel are mapped onto input image space. Then, at step **522**, the Jacobian matrix that approximates a non-uniform image scaling function in the mapped output pixel position is estimated. At step **524**, major and minor radii and angle of the major radius of an ellipse formed by row vectors of the Jacobian matrix are calculated. At step **526**, the radii of the ellipse are adjusted in accordance with required output image quality and processing speed. At step **528**, the three parameters of the ellipse are used in calculating three non-zero elements of matrix M, which represents a linear transformation from the ellipse to a circle with radius one. At step **530**, a footprint containing input image pixels is determined around the mapped coordinates of step **510**. The size and shape of this footprint depend on the chosen topology of the filtering and it is a trade-off between desired quality of the output image and computational power. At step **542** for each input pixel in the footprint, a distance between center of the ellipse and the input pixel position is calculated. Filtering coefficients for the inputs pixel from the footprint are calculated from a circularly symmetric profile at step **544** as a function of the calculated distances at the previous step. Finally, at step **550**, input pixels from the footprint are accumulated with weights determined at step **544**, and the generated output pixel is saved in output image at step **560**.

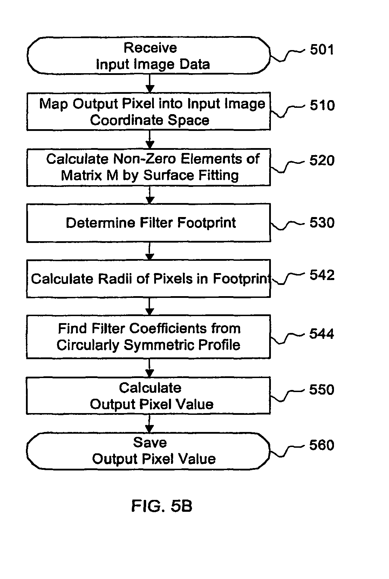

FIG. 5B shows a specific implementation of the flow diagram of FIG. 5A where the three non-zero elements of matrix M are calculated from polynomial fitted surfaces. In this example of the invention, three non-zero elements of the matrix M are calculated for a set of grid points and polynomial fitted with generating fitting coefficients. During digital image warping in each processed output pixel, the matrix elements of M are estimated from the fitting coefficients.

As will be apparent to those skilled in the art, various modifications and adaptations of the structure described above are possible without departing from the present invention, the scope of which is defined in the appended claims.

## Claims

1. A digital signal processing method for optimized hardware and software implementation of single-pass digital image resampling with an anisotropic filter to transform two-dimensional input image having an input pixel coordinate space and input digital pixel data, into an output image having an output pixel coordinate space and output digital pixel data, said method comprising:
(a) obtaining two-dimensional input digital image data for an output pixel;
(b) mapping the output pixel coordinates onto the input image coordinate space to determine a mapped coordinate position;
(c) determining the Jacobian matrix of the output pixel coordinates with respect to the input coordinates at the mapped coordinate position determined in (b);
(d) determining major (250) and minor radii (260) and the orientation of the major radius to uniquely determine an ellipse (240) around the mapped coordinate position determined in (b), formed by two row vectors of the determined Jacobian matrix in (c), wherein the major radius (250) of said ellipse (240) is aligned alongside an edge orientation, and the lengths of the radii depend on the strength of the edge;
(e) extending or reducing the radii of said ellipse to increase or reduce a filtering area;
(f) determining a footprint based on the result of (e) in the input image coordinate space around said mapped coordinate position determined in (b), to include input pixels participating in filtering;
(g) transforming the ellipse of (d) into a circle having a radius of one unit;
(h) determining coefficients of the anisotropic filter for all input pixels in the footprint from a circularly symmetric profile as a distance of each input pixel from a center of the circle having a radius of one unit;
(i) determining the value of the output pixel by accumulating values of the input pixels inside the footprint with coefficients determined in (h); and
(j) saving the value of the output pixel.

2. The method of claim 1, wherein the radii and the orientation of said ellipse of (d) are characterized via a 2x2 matrix, and wherein at least one element of said 2x2 matrix is forced to be zero.

3. The method of claim 2, wherein the values of non-zero elements of said 2x2 matrix in a subset of output pixels are pre-calculated and surface fitted, and wherein the matrix elements for an output pixel are evaluated from the fitting parameters.

4. The method of claim 1 wherein an elliptical footprint is used, and wherein the method uses a two-dimensional resampling filter with elliptical frequency response controlled by a matrix derived from said Jacobian matrix.

5. The method of claim 1 wherein a fixed-size rectangular footprint is used, and wherein the method uses a two-dimensional resampling filter with elliptical frequency response controlled by a matrix derived from said Jacobian matrix.

6. The method of claim 1, further combining profiles of sharpening and resampling components of the filter, to produce a detail enhanced output image.

7. The method of claim 1, wherein extending or reducing the radii of said ellipse comprises setting a predetermined maximum ratio for the radii of said ellipse.

8. A digital signal processing system for optimized hardware and software implementation of real-time single-pass digital image resampling with an anisotropic filter to transform two-dimensional input image having an input pixel coordinate space and input digital pixel data, into an output image having an output pixel coordinate space and output digital pixel data, said system comprising the following stages:
(a) an input interface for obtaining two-dimensional input digital image data for an output pixel;
(b) a coordinates generator (42) for mapping the output pixel coordinates onto the input image coordinate space to determine a mapped coordinate position;
(c) a local scale estimator (114), coupled to said input interface and said coordinate generator, for determining the Jacobian matrix of the output pixel coordinates with respect to the input coordinates at the mapped coordinate position;
(d) said local estimator, being further adapted for determining the major and minor radii and the orientation of the major radius to uniquely determine an ellipse around the mapped coordinate position, formed by two row vectors of the determined Jacobian matrix in (c), and align the major radius of said ellipse alongside an edge orientation and set the lengths of the radii based on a strength of the edge;
(e) said local scale estimator, being further adapted to extend or reduce the radii of said ellipse to increase or reduce a filtering area;
(f) a footprint generator (122), coupled to said local scale estimator and said coordinates generator, for determining a footprint in the input image coordinate space around the mapped coordinate position, to include input pixels participating in filtering;
(g) a filter coefficients generator (124), coupled to said footprint generator and said local scale estimator, to transform the ellipse of (d) into a circle having a radius of one unit;
(h) said filter coefficient generator, being further adapted to determine coefficients of the anisotropic filter for input pixels in the footprint from a circularly symmetric profile as a function of a distance of each input pixel from a center of the circle having a radius of one unit;
(i) a filter (126), coupled to said filter coefficient generator and said footprint generator, to determine the value of the output pixel by accumulating values of the input pixels inside the footprint with coefficients determined in (h); and
(j) an output interface, coupled to said filter, to save the value of the output pixel.

9. The system of claim 8, wherein said local scale estimator is adapted to characterize the radii and the orientation of said ellipse of (d) via a 2x2 matrix, and wherein at least one element of said 2x2 matrix is forced to be zero.

10. The system of claim 9, wherein the values of non-zero elements of said 2x2 matrix in a subset of output pixels are pre-calculated and surface fitted and said local scale estimator is adapted to evaluate the matrix elements for an output pixel from the fitting parameters.

11. The system of claim 8, wherein said footprint generator is adapted to use an elliptical footprint and said filter coefficients generator is adapted to use a two-dimensional resampling filter with elliptical frequency response controlled by a matrix derived from said Jacobian matrix.

12. The system of claim 8, wherein said footprint generator is adapted to use a fixed-size rectangular footprint and said filter coefficients generator is adapted to use a two-dimensional resampling filter with elliptical frequency response controlled by a matrix derived from said Jacobian matrix.

13. The system of claim 8, wherein said filter coefficients generator is adapted to combine profiles of sharpening and resampling components of the resampling filter to produce a detail enhanced output image.

14. The system of claim 1, wherein the local scale estimator extends or reduces the radii of said ellipse by setting a predetermined maximum bound, a predetermined minimum bound, and a predetermined maximum ratio for the radii of said ellipse.

## Patentansprüche

1. Verfahren zum Verarbeiten digitaler Signale für eine optimierte Hardware- und Software-Implementierung einer Einzeldurchlauf-Neuabtastung digitaler Bilder mit einem anisotropen Filter, um ein zweidimensionales Eingangsbild, das einen Eingangspixel-Koordinatenraum und digitale Eingangspixeldaten besitzt, in ein Ausgangsbild, das einen Ausgangspixel-Koordinatenraum und digitale Ausgangspixeldaten besitzt, zu transformieren, wobei das Verfahren umfasst:
(a) Erhalten zweidimensionaler digitaler Eingangsbilddaten für ein Ausgangspixel;
(b) Abbilden der Ausgangspixelkoordinaten auf den Eingangsbild-Koordinatenraum, um eine abgebildete Koordinatenposition zu bestimmen;
(c) Bestimmen der Jacobi-Matrix der Ausgangspixel-Koordinaten in Bezug auf die Eingangskoordinaten an der abgebildeten Koordinatenposition, die in (b) bestimmt worden ist;
(d) Bestimmen des Hauptradius (250) und des Nebenradius (260) sowie der Orientierung des Hauptradius, um eine Ellipse (240) um die in (b) bestimmte abgebildete Koordinatenposition, die durch zwei Zeilenvektoren der in (c) bestimmten Jacobi-Matrix gebildet wird, eindeutig zu bestimmen, wobei der Hauptradius (250) der Ellipse (240) auf eine Kantenorientierung ausgerichtet ist und die Längen der Radien von der Stärke der Kante abhängen;
(e) Verlängern oder Verkürzen der Radien der Ellipse, um einen Filterungsbereich zu vergrößern oder zu verkleinern;
(f) Bestimmen eines Bereichs bzw. Footprints anhand des Ergebnisses von (e) in dem Eingangsbild-Koordinatenraum um die in (b) bestimmte abgebildete Koordinatenposition, um Eingangspixel, die an der Filterung beteiligt sind, aufzunehmen;
(g) Transformieren der Ellipse von (d) in einen Kreis mit einem Radius einer Einheit;
(h) Bestimmen von Koeffizienten des anisotropen Filters für sämtliche Eingangspixel in dem Footprint aus einem kreisförmig symmetrischen Profil als einen Abstand jedes Eingangspixels von einem Zentrum des Kreises mit einem Radius einer Einheit;
(i) Bestimmen des Wertes des Ausgangspixels durch Akkumulieren von Werten der Eingangspixel innerhalb des Footprints mit in (h) bestimmten Koeffizienten; und
(j) Sichern des Wertes des Ausgangspixels.

2. Verfahren nach Anspruch 1, wobei die Radien und die Orientierung der Ellipse von (d) durch eine 2 x 2-Matrix gekennzeichnet sind und wobei wenigstens ein Element der 2 x 2-Matrix auf null gesetzt ist.

3. Verfahren nach Anspruch 2, wobei die Werte von von null verschiedenen Elementen der 2 x 2-Matrix in einer Untermenge von Ausgangspixeln im Voraus berechnet werden und an die Oberfläche angepasst werden und wobei die Matrixelemente für ein Ausgangspixel aus den Anpassungsparametern entwickelt werden.

4. Verfahren nach Anspruch 1, wobei ein elliptischer Fußabdruck verwendet wird und wobei das Verfahren ein zweidimensionales Neuabtastfilter mit elliptischer Frequenzantwort, das durch eine aus der Jacobi-Matrix abgeleitete Matrix gesteuert wird, verwendet.

5. Verfahren nach Anspruch 1, wobei ein rechteckiger Footprint mit fester Größe verwendet wird und wobei das Verfahren ein zweidimensionales Neuabtastfilter mit elliptischer Frequenzantwort, das durch eine aus der Jacobi-Matrix abgeleitete Matrix gesteuert wird, verwendet.

6. Verfahren nach Anspruch 1, das ferner Profile von Schärfungs- und Neuabtastkomponenten des Filters kombiniert, um ein in Einzelheiten verbessertes Ausgangsbild zu erzeugen.

7. Verfahren nach Anspruch 1, wobei das Vergrößern oder Verkleinern der Radien der Ellipse das Einstellen eines vorgegebenen maximalen Verhältnisses für die Radien der Ellipse umfasst.

8. System für die Verarbeitung digitaler Signale für eine optimierte Hardware- und Software-Implementierung einer Echtzeit-Einzeldurchlauf-Neuabtastung digitaler Bilder mit einem anisotropen Filter, um ein zweidimensionales Eingangsbild, das einen Eingangspixel-Koordinatenraum und digitale Eingangspixeldaten besitzt, in ein Ausgangsbild, das einen Ausgangspixel-Koordinatenraum und digitale Ausgangspixeldaten besitzt, zu transformieren, wobei das System die folgenden Stufen aufweist:
(a) eine Eingangsschnittstelle, um zweidimensionale digitale Eingangsbilddaten für ein Ausgangspixel zu erhalten;
(b) einen Koordinatengenerator (42), um die Ausgangspixel-Koordinaten auf den Eingangsbild-Koordinatenraum abzubilden, um eine abgebildete Koordinatenposition zu bestimmen;
(c) eine Lokalskala-Schätzeinrichtung (114), die mit der Eingangsschnittstelle und mit dem Koordinatengenerator gekoppelt ist, um die Jacobi-Matrix der Ausgangspixel-Koordinaten in Bezug auf die Eingangskoordinaten der abgebildeten Koordinatenposition zu bestimmen;
(d) wobei die lokale Schätzeinrichtung ferner dazu ausgelegt ist, den Haupt- und den Nebenradius sowie die Orientierung des Hauptradius zu bestimmen, um eine Ellipse um die abgebildete Koordinatenposition, die durch zwei Zeilenvektoren der in (c) bestimmten Jacobi-Matrix gebildet wird, eindeutig zu bestimmen und den Hauptradius der Ellipse auf eine Kantenorientierung auszurichten und die Längen der Radien anhand einer Stärke der Kante festzulegen;
(e) wobei die Lokalskala-Schätzeinrichtung ferner dazu ausgelegt ist, die Radien der Ellipse zu verlängern oder zu verkürzen, um einen Filterungsbereich zu vergrößern oder zu verkleinern;
(f) einen Bereichs- bzw. Footprintgenerator (122), der mit der Lokalskala-Schätzeinrichtung und mit dem Koordinatengenerator gekoppelt ist, um einen Bereich bzw. Footprint in dem Eingangsbild-Koordinatenraum um die abgebildete Koordinatenposition zu bestimmen, um Eingangspixel, die an der Filterung beteiligt sind, aufzunehmen;
(g) einen Filterkoeffizienten-Generator (124), der mit dem Fußabdruckgenerator und mit der Lokalskala-Schätzeinrichtung gekoppelt ist, um die Ellipse von (d) in einen Kreis mit einem Radius einer Einheit zu transformieren;
(h) wobei der Filterkoeffizienten-Generator ferner dazu ausgelegt ist, Koeffizienten des anisotropen Filters für Eingangspixel in dem Footprint aus einem kreisförmigen symmetrischen Profil als eine Funktion eines Abstands jedes Eingangspixel von einem Zentrum des Kreises mit einem Radius einer Einheit zu bestimmen;
(i) ein Filter (126), das mit dem Filterkoeffizienten-Generator und mit dem Fußabdruckgenerator gekoppelt ist, um den Wert des Ausgangspixels durch Akkumulieren von Werten der Eingangspixel innerhalb des Footprints mit in (h) bestimmten Koeffizienten zu akkumulieren; und
(j) eine Ausgangsschnittstelle, die mit dem Filter gekoppelt ist, um den Wert des Ausgangspixels zu sichern.

9. System nach Anspruch 8, wobei die Lokalskala-Schätzeinrichtung dazu ausgelegt ist, die Radien und die Orientierung der Ellipse von (d) durch ein 2 x 2-Matrix zu kennzeichnen und wobei wenigstens ein Element der 2 x 2-Matrix auf null gesetzt ist.

10. System nach Anspruch 9, wobei die Werte von von null verschiedenen Elementen der 2 x 2-Matrix in einer Untermenge von Ausgangspixeln im Voraus berechnet werden und an die Oberfläche angepasst werden und wobei die Lokalskala-Schätzeinrichtung dazu ausgelegt ist, die Matrixelemente für ein Ausgangspixel aus den Anpassungsparametern zu entwickeln.

11. System nach Anspruch 8, wobei der Fußabdruckgenerator dazu ausgelegt ist, einen elliptischen Footprint zu verwenden, und der Filterkoeffizienten-Generator dazu ausgelegt ist, ein zweidimensionales Neuabtastfilter mit elliptischer Frequenzantwort, das durch eine von der Jacobi-Matrix abgeleitete Matrix gesteuert wird, zu verwenden.

12. System nach Anspruch 8, wobei der Fußabdruckgenerator dazu ausgelegt ist, einen rechtwinkligen Footprint mit fester Größe zu verwenden und der Filterkoeffizienten-Generator dazu ausgelegt ist, ein zweidimensionales Neuabtastfilter mit elliptischer Frequenzantwort, das durch eine von der Jacobi-Matrix abgeleitete Matrix gesteuert wird, zu verwenden.

13. System nach Anspruch 8, wobei der Filterkoeffizienten-Generator dazu ausgelegt ist, Profile von Schärfungs- und Neuabtastungskomponenten des Neuabtastfilters zu kombinieren, um ein in Einzelheiten verbessertes Ausgangsbild zu erzeugen.

14. System nach Anspruch 1, wobei die Lokalskala-Schätzeinrichtung die Radien der Ellipse durch Einstellen einer vorgegebenen maximalen Schranke, einer vorgegebenen minimalen Schranke und eines vorgegebenen maximalen Verhältnisses für die Radien der Ellipse verlängert oder verkürzt.

## Revendications

1. Procédé de traitement de signal numérique pour une mise en oeuvre matérielle et logicielle optimisée d'un rééchantillonnage d'image numérique en une seule passe avec un filtre anisotrope pour transformer une image d'entrée bidimensionnelle ayant un espace de coordonnées de pixels d'entrée et des données de pixels numériques d'entrée, en une image de sortie ayant un espace de coordonnées de pixels de sortie et des données de pixels numériques de sortie, ledit procédé comportant les étapes consistant à :
(a) obtenir des données d'image numérique d'entrée bidimensionnelle pour un pixel de sortie ;
(b) mapper les coordonnées de pixel de sortie dans l'espace de coordonnées d'image d'entrée pour déterminer une position de coordonnées mappées ;
(c) déterminer la matrice jacobienne des coordonnées de pixel de sortie par rapport aux coordonnées d'entrée à la position de coordonnées mappées déterminée en (b) ;
(d) déterminer des rayons majeur (250) et mineur (260) et l'orientation du rayon majeur pour déterminer de manière unique une ellipse (240) autour de la position de coordonnées mappées déterminée en (b), formée par deux vecteurs lignes de la matrice jacobienne déterminée en (c), dans lequel le rayon majeur (250) de ladite ellipse (240) est aligné le long d'une orientation de bord, et les longueurs des rayons dépendent de la résistance du bord ;
(e) étendre ou réduire les rayons de ladite ellipse pour augmenter ou réduire une aire de filtrage ;
(f) déterminer une empreinte sur la base du résultat de (e) dans l'espace de coordonnées d'image d'entrée autour de ladite position de coordonnées mappées déterminée en (b), pour inclure des pixels d'entrée participant au filtrage ;
(g) transformer l'ellipse de (d) en un cercle ayant un rayon d'une unité ;
(h) déterminer des coefficients du filtre anisotrope pour tous les pixels d'entrée dans l'empreinte à partir d'un profil circulairement symétrique comme une distance de chaque pixel d'entrée par rapport à un centre du cercle ayant un rayon d'une unité ;
(i) déterminer la valeur du pixel de sortie en cumulant des valeurs des pixels d'entrée à l'intérieur de l'empreinte avec des coefficients déterminés en (h) ; et
(j) enregistrer la valeur du pixel de sortie.

2. Procédé selon la revendication 1, dans lequel les rayons et l'orientation de ladite ellipse de (d) sont caractérisés via une matrice 2x2, et dans lequel au moins un élément de ladite matrice 2x2 est forcé pour être égal à zéro.

3. Procédé selon la revendication 2, dans lequel les valeurs des éléments non nuls de ladite matrice 2x2 dans un sous-ensemble de pixels de sortie sont précalculées et ajustées en termes de surface, et dans lequel les éléments de matrice pour un pixel de sortie sont évalués à partir des paramètres d'ajustement.

4. Procédé selon la revendication 1, dans lequel une empreinte elliptique est utilisée, et dans lequel le procédé utilise un filtre de rééchantillonnage bidimensionnel avec une réponse en fréquence elliptique commandée par une matrice dérivée de ladite matrice jacobienne.

5. Procédé selon la revendication 1, dans lequel une empreinte rectangulaire de taille fixe est utilisée, et dans lequel le procédé utilise un filtre de rééchantillonnage bidimensionnel avec une réponse en fréquence elliptique commandée par une matrice dérivée de ladite matrice jacobienne.

6. Procédé selon la revendication 1, combinant en outre des profils de composants d'accentuation de contour et de rééchantillonnage du filtre, pour produire une image de sortie améliorée en termes de détail.

7. Procédé selon la revendication 1, dans lequel l'extension ou la réduction des rayons de ladite ellipse comporte l'établissement d'un rapport maximal prédéterminé pour les rayons de ladite ellipse.

8. Système de traitement de signal numérique pour une mise en oeuvre matérielle et logicielle optimisée d'un rééchantillonnage d'image numérique en une seule passe en temps réel avec un filtre anisotrope pour transformer une image d'entrée bidimensionnelle ayant un espace de coordonnées de pixels d'entrée et des données de pixels numériques d'entrée, en une image de sortie ayant un espace de coordonnées de pixels de sortie et des données de pixels numériques de sortie, ledit système comportant les étages suivants :
(a) une interface d'entrée pour obtenir des données d'image numérique d'entrée bidimensionnelle pour un pixel de sortie ;
(b) un générateur de coordonnées (42) pour mapper les coordonnées de pixel de sortie dans l'espace de coordonnées d'image d'entrée pour déterminer une position de coordonnées mappées ;
(c) un estimateur d'échelle local (114), couplé à ladite interface d'entrée et audit générateur de coordonnées, pour déterminer la matrice jacobienne des coordonnées de pixel de sortie par rapport aux coordonnées d'entrée à la position de coordonnées mappées ;
(d) ledit estimateur local étant en outre adapté pour déterminer les rayons majeur et mineur et l'orientation du rayon majeur pour déterminer de manière unique une ellipse autour de la position de coordonnées mappées, formée par deux vecteurs lignes de la matrice jacobienne déterminée en (c), et aligner le rayon majeur de ladite ellipse le long d'une orientation de bord et fixer les longueurs des rayons sur la base d'une résistance du bord ;
(e) ledit estimateur d'échelle local étant en outre adapté pour étendre ou réduire les rayons de ladite ellipse pour augmenter ou réduire une aire de filtrage ;
(f) un générateur d'empreinte (122), couplé audit estimateur d'échelle local et audit générateur de coordonnées, pour déterminer une empreinte dans l'espace de coordonnées d'image d'entrée autour de la position de coordonnées mappées, pour inclure des pixels d'entrée participant au filtrage ;
(g) un générateur de coefficients de filtre (124), couplé audit générateur d'empreinte et audit estimateur d'échelle local, pour transformer l'ellipse de (d) en un cercle ayant un rayon d'une unité ;
(h) ledit générateur de coefficients de filtre étant en outre adapté pour déterminer des coefficients du filtre anisotrope pour des pixels d'entrée dans l'empreinte à partir d'un profil circulairement symétrique en fonction d'une distance de chaque pixel d'entrée par rapport à un centre du cercle ayant un rayon d'une unité ;
(i) un filtre (126), couplé audit générateur de coefficients de filtre et audit générateur d'empreinte, pour déterminer la valeur du pixel de sortie en cumulant des valeurs des pixels d'entrée à l'intérieur de l'empreinte avec les coefficients déterminés en (h) ; et
(j) une interface de sortie, couplée audit filtre, pour enregistrer la valeur du pixel de sortie.

9. Système selon la revendication 8, dans lequel ledit estimateur d'échelle local est adapté pour caractériser les rayons et l'orientation de ladite ellipse de (d) via une matrice 2x2, et dans lequel au moins un élément de ladite matrice 2x2 est forcé pour être égal à zéro.

10. Système selon la revendication 9, dans lequel les valeurs des éléments non nuls de ladite matrice 2x2 dans un sous-ensemble de pixels de sortie sont précalculées et ajustées en termes de surface et ledit estimateur d'échelle local est adapté pour évaluer les éléments de matrice pour un pixel de sortie à partir des paramètres d'ajustement.

11. Système selon la revendication 8, dans lequel ledit générateur d'empreinte est adapté pour utiliser une empreinte elliptique et ledit générateur de coefficients de filtre est adapté pour utiliser un filtre de rééchantillonnage bidimensionnel avec une réponse en fréquence elliptique commandée par une matrice dérivée de ladite matrice jacobienne.

12. Système selon la revendication 8, dans lequel ledit générateur d'empreinte est adapté pour utiliser une empreinte rectangulaire de taille fixe et ledit générateur de coefficients de filtre est adapté pour utiliser un filtre de rééchantillonnage bidimensionnel avec une réponse en fréquence elliptique commandée par une matrice dérivée de ladite matrice jacobienne.

13. Système selon la revendication 8, dans lequel ledit générateur de coefficients de filtre est adapté pour combiner des profils de composants d'accentuation de contour et de rééchantillonnage du filtre de rééchantillonnage pour produire une image de sortie améliorée en termes de détail.

14. Système selon la revendication 1, dans lequel l'estimateur d'échelle local étend ou réduit les rayons de ladite ellipse en fixant une borne maximale prédéterminée, une borne minimale prédéterminée, et un rapport maximal prédéterminé pour les rayons de ladite ellipse.
